(19) Europäisches Patentamt
European Patent Office
Office européen des brevets

(11) Veröffentlichungsnummer: **0 101 898**
**A1**

(12) # EUROPÄISCHE PATENTANMELDUNG

(21) Anmeldenummer: **83107175.8**

(22) Anmeldetag: **22.07.83**

(51) Int. Cl.³: **C 07 D 279/20**
C 07 D 279/16, C 07 D 513/04
A 61 K 31/54
//(C07D513/04, 333/00, 279/00),
(C07D513/04, 307/00, 279/00)

(30) Priorität: **04.08.82 DE 3229122**

(43) Veröffentlichungstag der Anmeldung:
**07.03.84 Patentblatt 84/10**

(84) Benannte Vertragsstaaten:
**AT BE CH DE FR GB IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Niemers, Ekkehard, Dr.**
**In den Birken 51a**
**D-5600 Wuppertal 1(DE)**

(72) Erfinder: **Grützmann, Rudi, Dr.**
**Helsinkistrasse 20**
**D-5650 Solingen 1(DE)**

(72) Erfinder: **Mardin, Mithat, Dr.**
**Untere Bergerheide 15**
**D-5600 Wuppertal 1(DE)**

(72) Erfinder: **Busse, Wolf-Dieter, Dr.**
**c/o 400 Morgan Lane**
**West Haven, Ct 06516(US)**

(72) Erfinder: **Meyer, Horst, Dr.**
**Henselweg 11**
**D-5600 Wuppertal 1(DE)**

(54) **Anellierte 4H-1,4-Benzothiazine, Verfahren zu ihrer Herstellung und ihre Verwendung als Arzneimittel.**

(57) Anellierte 4H-1,4- Benzothiazine der Formel I

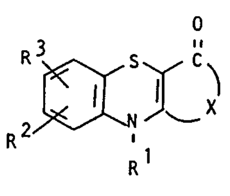

I

worin

$R_1$ für Wasserstoff und gegebenenfalls substituiertes Alkyl steht,

$R^2$, $R^3$ für Wasserstoff, gegebenenfalls substituiertes Alkyl, Halogen, Nitro, Cyano, Alkylsulfonyl, Arylsulfonyl, Hydroxy, Alkoxy, Acyloxy stehen und $R^2$ und $R^3$ gleich oder verschieden sein können, mit der Einschränkung, daß $R^2$ oder $R^3$ nicht Brom, oder $R^2$ und $R^3$ nicht gleichzeitig Wasserstoff bedeuten,

X für Alkylen (mit 2 bis 4 C-Atomen), das einfach oder mehrfach durch Alkyl, Alkoxy, Aryl, Fluor oder Alkoxycarbonyl, eine Methylengruppe der Alkylenbrücke kann auch durch O, S, $SO_2$, SO, NH oder N-Alkyl ersetzt sein, substituiert sein kann,

steht.

Verfahren zu ihrer Herstellung, ihre Verwendung als Arzneimittel, insbesondere ihre Verwendung als Lipoxygenasehemmer, diese enthaltende Arzneimittel und deren Herstellung.

EP 0 101 898 A1

Croydon Printing Company Ltd

BAYER AKTIENGESELLSCHAFT 5090 Leverkusen, Bayerwerk

Zentralbereich
Patente, Marken und Lizenzen Je/ABc

# Anellierte 4H-1,4-Benzothiazine, Verfahren zu ihrer Herstellung und ihre Verwendung als Arzneimittel

Die vorliegende Erfindung betrifft anellierte 4H-1,4-Benzothiazine, mehrere Verfahren zu ihrer Herstellung, ihre Verwendung als Arzneimittel, insbesondere ihre Verwendung als Lipoxygenasehemmer, diese enthaltende Arzneimittel und deren Herstellung.

Es ist bekannt, daß die durch das Enzym Lipoxygenase gebildeten Metaboliten der Arachidonsäure-Leukotriene und slow reacting Substance of anaphylaxis (SRS-A)- an der Entstehung von entzündlichen und allergischen Prozessen beteiligt sind, vgl. z.B. E.J. Goetzl, Immunology 40 709 (1980) und Medical Clinics of North America 65 , 809 (1981).

Bekannte Lipoxygenasehemmer wie Nordihydroguaretsäure, 3-Amino-1-(3-trifluormethylphenyl)-pyrazolin, Phenidon und 5,8,11,14-Eikosatetraensäure sind entweder gleichzeitig als Cyclooxygenasehemmer oder erst bei sehr hohen Konzentrationen wirksam. Die Hemmung des Enzyms Cyclooxygenase

des Arachidonsäuremetabolismus führt zu einer globalen Prostaglandinsynthesehemmung und zu einer Stimulation des Lipoxygenaseweges, was eine Gastrotoxizität bzw. pro-inflammatorische und asthmatische Wirkungen verursacht. Außerdem haben schon bekannte Lipoxygenasehemmer wie 3-Amino-1-(m-trifluormethylphenyl)pyrazolin -2 bei systemischer Verabreichung (z.B. oral) toxische Nebenwirkungen. Es besteht deshalb ein Bedarf nach oral wirksamen Verbindungen, die diese unerwünschten Nebenwirkungen nicht besitzen.

Überraschenderweise hemmen die erfindungsgemäßen anellierten 4H-1,4-Benzothiazine die Lipoxygenase bereits in solcher Konzentration, bei denen die Cyclooxygenase wenig beeinflußt wird.

Die erfindungsgemäßen Verbindungen stimulieren überraschenderweise auch die Synthese von Prostacyclin in arteriellen Gefäßen in vitro, möglicherweise als Folge ihrer lipoxygenase-hemmenden Eigenschaft. Bezüglich dieser Wirkung sind die Verbindungen stärker wirksam als der genannte Lipoxygenasehemmer 3-Amino-1-(m-trifluormethylphenyl)-pyrazolin-2. (Proc. of British Pharmacological Society 920 P (1981). Die erfindungsgemäßen Verbindungen stimulieren auch an Kaninchenaortastreifen die Prostacyclin-Synthese.

Die erfindungsgemäßen Verbindungen besitzen auch eine antiphlogistische Wirkung im Carragenan-induzierten Ödemmodell, wenn sie systemisch, besonders oral, und lokal, besonders cutan, verabreicht werden. Sie besitzen ebenfalls antimetastatische Wirkung.

Die erfindungsgemäßen, Lipoxygenase-hemmenden anellierten 4H-1,4-Benzothiazine sind somit als Arzneimittel bei der Behandlung von entzündlichen, allergischen und kardiovaskulären Erkrankungen einsetzbar. Sie können insbesondere als Antiphlogistika, Antirheumatika, Antiatherosklerotika, Antithrombotika, Antiarthrotika, Antiasthmatika, Antiallergika, Antimetastatika und Gastroprotektiva Verwendung finden.

Weitere Anwendungsgebiete sind die Behandlung und Verhütung cerebral-ischämischer, transitorisch ischämischer Anfälle, Myocardinfarkten, Schlaganfällen und Arrhythmien. Die Verbindungen eignen sich auch zur Behandlung peripherer Gefäßkrankheiten und Durchblutungsstörungen beim Menschen.

Gegenstand der vorliegenden Erfindung sind daher Verbindungen der nachstehend definierten Formel I; sowie die nachstehend aufgeführten Verfahren zur Herstellung von Verbindungen gemäß Formel I; sowie Verbindungen der allgemeinen Formel I zur Verwendung bei der Bekämpfung von Erkrankungen, insbesondere als Lipoxygenasehemmer, Antiphlogistika und/oder Antimetastatika; sowie Arzneimittel, enthaltend mindestens eine Verbindung der allgemeinen Formel I; sowie die Verwendung von Verbindungen der allgemeinen Formel I zur Bekämpfung von Krankheiten insbesondere als Lipoxygenasehemmer, Antiphlogistika und/oder Antimetastatika; sowie Verfahren zur Herstellung von Arzneimitteln enthaltend mindestens eine Verbindung der Formel I, indem man Verbindungen der allgemeinen Formel I gegebenenfalls unter Verwendung von üblichen Hilfs- und Trägerstoffen in eine geeignete Applikationsform überführt.

Gegenstand der vorliegenden Erfindung sind anellierte 4H-1,4-Benzothiazine der allgemeinen Formel I

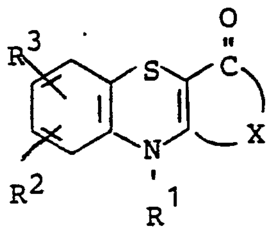

I

worin

$R^1$ für Wasserstoff und gegebenenfalls substituiertes Alkyl steht,

$R^2$, $R^3$ für Wasserstoff, gegebenfalls substituiertes Alkyl, Halogen, Nitro, Cyano, Alkylsulfonyl, Arylsulfonyl, Hydroxy, Alkoxy, Acyloxy stehen und

$R^2$ und $R^3$ gleich oder verschieden sein können, wobei $R^2$ oder $R^3$ jedoch nicht Brom oder $R^2$ und $R^3$ nicht gleichzeitig Wasserstoff bedeuten,

X für Alkylen (mit 2 bis 4 C-Atomen), das ein- oder mehrfach substituiert sein kann durch Alkyl, Alkoxy, Aryl, Fluor oder Alkoxycarbonyl, eine Methylengruppe der Alkylenbrücke kann auch durch O, S, SO, $SO_2$, NH oder N-Alkyl ersetzt sein,

steht.

Ein weiterer Gegenstand der vorliegenden Erfindung sind anellierte 4H-1,4-Benzothiazine der allgemeinen Formel I

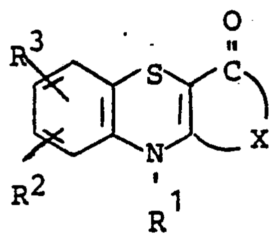

I

worin

$R_1$ für Wasserstoff und gegebenenfalls substituiertes Alkyl steht,

$R_2$, $R_3$ für Wasserstoff, gegebenenfalls substituiertes Alkyl, Halogen, Nitro, Cyano, Alkylsulfonyl, Arylsulfonyl, Hydroxy, Alkoxy, Acyloxy und

X für Alkylen (mit 2 bis 4 C-Atomen), das einfach oder mehrfach durch Alkyl, Alkoxy, Aryl, Fluor oder Alkoxycarbonyl substituiert sein kann, eine Methylengruppe der Alkylenbrücke kann auch durch O, S, $SO_2$, SO, NH oder N-Alkyl ersetzt sein,

steht, zur Verwendung bei der Bekämpfung von Krankheiten, insbesondere als Lipoxygenasehemmer, Antiphlogistika und/oder Antimetastatika; ferner enthaltend in Arzneimitteln; sowie die Verwendung dieser 4H-1,4-Benzothiazine bei der Bekämpfung von Krankheiten.

In der allgemeinen Formel I steht gegebenenfalls substituiertes Alkyl, $R^1$, $R^2$, $R^3$, für geradkettiges oder verzweigtes Alkyl mit vorzugsweise 1 bis 8, insbesondere 1 bis 4 Kohlenstoffatomen. Beispielhaft seien gegebenenfalls substituiertes Methyl, Ethyl, n- und i-Propyl, n-, i- und t-Butyl genannt.

Die Alkylreste $R^1$ bis $R^3$ können 1- bis 3-mal, vorzugsweise einmal , durch folgende Substituenten substituiert sein: Halogen, Dialkylamino, Pyrrolidino, Piperidino, gegebenenfalls substituiertes Aryl, Alkoxy, Alkylthio, Alkylsulfinyl, Alkylsulfonyl, $-CO-R^4$, wobei $R^4$ für Alkyl, gegebenenfalls substituiertes Aryl, Hydroxy, Alkoxy, Amino, Alkylamino, Dialkylamino, Pyrrolidino, Piperidino, Morpholino steht.

Als gegebenenfalls substituiertes Aryl $R^4$ und Substituent der Alkylreste $R^1$ bis $R^3$ steht Aryl mit vorzugsweise 6 bis 20 Kohlenstoffatomen im Arylteil. Beispielhaft seinen Phenyl oder Naphthyl genannt. Die Arylreste können 1- bis 3-mal, vorzugsweise einmal in o-, m- oder p-Stellung durch folgende Substituenten substituiert sein: Halogen, Alkyl, Alkoxy, Trifluormethyl, Nitro.

Als gegebenenfalls substituiertes Alkoxy $R^2$, $R^3$ und als Substituent von X steht geradkettiges oder verzweigtes Alkoxy mit vorzugsweise 1 bis 8, insbesondere 1 bis 4 Kohlenstoffatomen. Die Alkoxyreste können 1- bis 3-mal, vorzugsweise einmal, durch folgende Substituenten substituiert sein: Halogen, insbesondere Fluor und Chlor, Aryl (gegebenenfalls subst.), Hydroxy, Alkoxy, Alkylthio, Cyano.

Die erfindungsgemäßen Stoffe der allgemeinen Formel I lassen sich herstellen, indem man 2-Aminothiophenole der Formel II mit Verbindungen der Formel III in Dimethylsulfoxid zur Reaktion bringt (vgl. J. Chem. Soc. Perkin Trans. I, 1976, 1146-49).

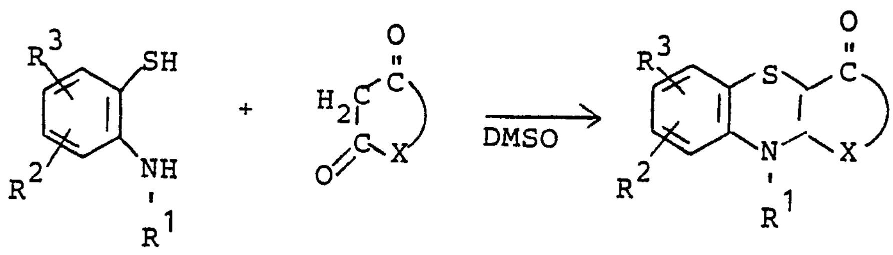


II III I

Die Verbindungen der Formeln II und III sind entweder literaturbekannt oder lassen sich in Analogie zu literaturbekannten Verbindungen herstellen. Die Reaktionstemperatur kann zwischen 50°C und 200°C liegen, vorzugsweise zwischen 100°C und 150°C. In einigen Fällen ist es zweckmäßig unter Sauerstoffausschluß zu arbeiten.

Die erfindungsgemäßen Stoffe lassen sich ferner herstellen, indem man Disulfide der Formel IV mit Dicarbonylverbindungen der Formel III zur Reaktion bringt /vergl. Phosphorus and Sulfur 3, 309-314, (1977)7.

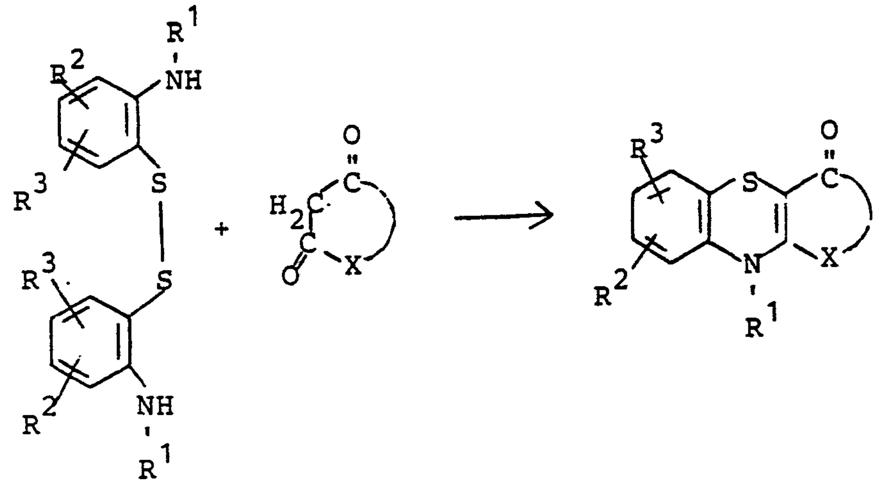

IV III I

Die Reaktion wird durch Säuren (z.B. p-Toluolsulfonsäre) katalysiert.

Als Lösungsmittel kommen in Frage: Benzol, Toluol, Pyridin, DMSO und DMF.

Die Reaktionstemperatur kann zwischen 20°C und 180°C liegen, vorzugsweise zwischen 35°C und 150°C.

Es ist zweckmäßig unter Sauerstoffausschluß zu arbeiten.

Die Reaktion wird meist mit äquimolekularen Mengen der Ausgangsmaterialien durchgeführt, gelegentlich wird auch ein Überschuß (bis zu 110 %) an Dicarbonylverbindung verwendet.

Erfindungsgemäße Stoffe der Formel I, bei denen $R^1$ für Alkyl (gegebenenfalls substituiertes) steht, lassen sich aus den entsprechenden Verbindungen, bei denen R' für

H steht, nach den üblichen Alkylierungsmethoden herstellen, z.B. durch Umsetzung mit $CH_3J$, $C_2H_5$-Br oder ω-Bromacetophenon.

Der Nachweis der lipoxygenasehemmenden Eigenschaften der erfindungsgemäßen Verbindungen, erfolgt analog der Methode von Bailey et al., Journal of Biol. Chemistry 255, 5996, (1980) und nach Blackwell und Flower, Prostaglandins 16, 417 (1978). Bei dieser Testmethode wird der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschicht-Scanner ausgewertet. Bei diesen Testbedingungen werden die markierten Metaboliten von der nicht umgesetzten Arachidonsäure getrennt und sind anschließend quantitativ auswertbar. Die Verteilung der Radioaktivität auf die während der Metabolisierung gebildeten Cyclooxygenaseprodukte Thromboxan $B_2$ ($TXB_2$) und 12-Hydroxy-5,8,10-heptadekatriensäure (HHT) bzw. das Lipoxygenaseprodukt 12-Hydroxy-5,8,11,14-eikosatetraensäure (HETE) unter dem Einfluß der Inhibitoren stellt ein Maß für die Hemmung der Enzyme dar.

Die Lipoxygenasehemmung der erfindungsgemäßen Verbindungen läßt sich an der Hemmung der HETE-Synthese messen. Es zeigt sich, daß die Synthese von $TXB_2$ und von HHT unbeeinflußt bleibt, während der Arachidonsäureumsatz abnimmt. Wie aus der nachfolgenden Tabelle ersichtlich ist, bewirken die erfindungsgemäßen Verbindungen eine signifikante Hemmung der Plättchenlipoxygenase (HETE-Synthese). (Vergl. Tab. 1)

Analog dem o.a. Text lassen sich die lipoxygenasehemmenden Eigenschaften der erfindungsgemäßen Verbindungen auch an Leukozyten nachweisen.

Die polymorphkernigen Leukozyten des Menschen und des Kaninchens metabolisieren die Arachidonsäure zu 5-Hydroxy-5,8,11,14-eikosatetraensäure (5-HETE) und Leukotrien $B_4$ (5S, 12R-Dihydroxy-6 cis, 8,10-trans-14 ciseikosatetraensäure). Die Hemmung der Freisetzung von 5-HETE und Leukotrien $B_4$ aus den Leukozyten stellt ein Maß für den lipoxygenasehemmenden Effekt der erfindungsgemäßen Verbindungen dar. (Vergl. Tab. 1).

Der Test mit den Humanleukozyten wird nach Borgeat und Samuelsson (j. Biol. Chem. 254; 2643, 1979 und Proc. Natl. Acad. Sci. USA, 76, 2148 1979), mit Kaninchenleukozyten nach Walker und Parish (Inter. Archs. Allergy appl. Immun. 66, 83, 1981) durchgeführt.

Der Nachweis der Prostacyclin-stimulierenden Wirkung erfolgte durch Bestimmung der Freisetzung von Prostacyclin nach einstündiger Inkubation von Kaninchenaortenstreifen mit den erfindungsgemäßen Verbindungen (analog nach Moncada et al., Lancet 1977, I, 18) und anschließender radioimmunologischer Bestimmung des stabilen Prostacyclinmetaboliten 6-Keto-PGF 1 (B.M. Peskar et al. FEBS Letters 121, 25, 1980).

Die erfindungsgemäßen Verbindungen sind auch in vivo wirksam. Diese Wirkung wird durch die Messung der Hemmung der Leukozytenmigration nach an sich bekannten Methoden nachgewiesen (vgl. Higgs et al., Biochemical Pharmacology 28, 1959, (1979) und Eur. J. Pharmacol. 66, 81 (1981)).

Die neuen Wirkstoffe können in bekannter Weise in die üblichen Formulierungen, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel überführt werden. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuß-/Sesamöl), Alkohole (z.B. Ethylalkohol, Glycerin), Glykole (z.B. Propylenglykol, Polyethylenglykol), N-Alkylpyrrolidone, feste Trägerstoffe, wie z.B. natürliche Gesteinsmehle (z.B. Kaoline, Tonerden, Talkum, Kreide),

synthetische Gesteinsmehle (z.B. hochdisperse Kieselsäure, Silikate), Zucker (z.B. Rohr-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere cutan. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/ oder Elixieren, die für orale Anwendungen gedacht sind, können die Wirkstoffe außer mit den obengenannten Hilfsstoffen mit verschiedenen Geschmacksaufbesserern oder gefärbten bzw. farbgebenden Stoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10

mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht bzw. der Art des Applikationsweges, aber auch aufgrund der Art und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Die obigen Ausführungen gelten für die Applikation sowohl in der Human- als auch in der Tiermedizin in sinngemäß gleicher Weise.

Die folgenden Beispiele sollen die Erfindung näher erläutern: (siehe auch Tabelle 1).

Beispiel 1

7-Ethoxy-1-oxo-1,2,3,4-tetrahydrophenothiazin

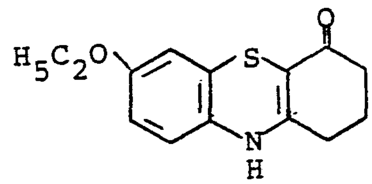

Eine Lösung von 5,4 g (0,016 Mol) 4,4'-Diethoxy-2,2'-dithiodianilin und 3,6 g (0,032 Mol) 1,3-Cyclohexandion in 20 ml Dimethylsulfoxid wurde eine Stunde auf 110 - 120°C erhitzt. Nach dem Erkalten wurde das Gemisch auf 100 ml Wasser gegeben und mit Aceton versetzt. Es bildete sich ein Niederschlag, der abgesaugt und aus DMF/Isopropanol umkristallisiert wurde.
Fp. 201 - 202°C
Ausbeute 5 g ( 59 % der Theorie).

In gleicher Weise lassen sich folgende Präparate herstellen:

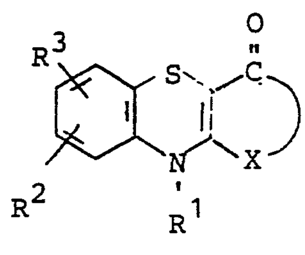

| Beispiel | X | $R^1$ | $R^2$ | $R^3$ | Fp |
|---|---|---|---|---|---|
| 2 | $-CH_2-CH_2-CH_2-$ | H | H | H | 253°C |
| 3 | $-CH_2-CH_2-CH_2-$ | $CH_3$ | H | H | |
| 4 | $-CH_2-CH_2-CH_2-$ | H | $7-CH_3$ | H | 231°C |
| 5 | $-CH_2-CH_2-CH_2-$ | H | 7-Cl | H | 250°C |
| 6 | 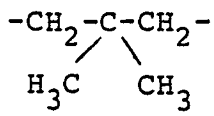 | H | H | H | 270°C |
| 7 | 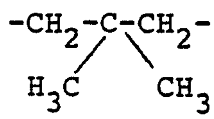 | $CH_3$ | H | H | 153°C |
| 8 | $-CH_2-CH_2-$ | H | H | H | |
| 9 | $-(CH_2)_4-$ | H | H | H | |
| 10 | $-CH_2-SO_2-CH_2-$ | H | H | H | |
| 11 | $-CH_2-CH_2-CH_2-$ | H | $5-CH_3$ | $7-CH_3$ | |
| 12 | $-CH_2-CH_2-CH_2-$ | H | 7-CN | H | |
| 13 | 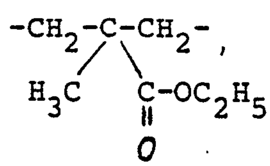 | H | H | H | |
| 14 | 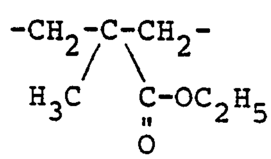 | H | H | H | |
| 15 | $-CH_2-O-CH_2-$ | H | H | H | |

| Beispiel | X | $R^1$ | $R^2$ | $R^3$ | Fp |
|---|---|---|---|---|---|
| 16 | $-CH_2-CH_2-CH_2-$ | H | $7-NO_2$ | H | |
| 17 | $-CH_2-CH_2-CH_2-$ | $CH_2-\overset{O}{\overset{\|}{C}}-Ph$ | H | H | |
| 18 | -CH2$-CH_2-CH_2-$ | H | $7-CH_3SO_2$ | H | |
| 19 | -CH2$-CH_2-CH_2-$ | $C_2H_5$ | $7-CH_3$ | H | |
| 20 | $-CH_2-CH_2-CH_2-$ | H | $7-OCH_3$ | H | 213°C |

Tabelle 1

| Substanz Beispiel | Minimale Konzentration für die Hemmung der Lipoxygenase in Kaninchen-PMN-Leukozyten in g/ml |
|---|---|
| 1 | $5 \times 10^{-7}$ bis $10^{-6}$ |
| 2 | $5 \times 10^{-7}$ bis $10^{-6}$ |
| 4 | $5 \times 10^{-7}$ bis $10^{-6}$ |
| 5 | $5 \times 10^{-7}$ bis $10^{-6}$ |
| 8 | $5 \times 10^{-7}$ bis $10^{-6}$ |
| 20 | $5 \times 10^{-7}$ bis $10^{-6}$ |

Patentansprüche

1. Anellierte 4H-1,4-Benzothiazine der allgemeinen Formel I

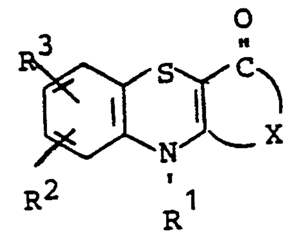

I

worin

$R^1$ für Wasserstoff und gegebenenfalls substituiertes Alkyl steht,

$R^2$, $R^3$ für Wasserstoff, gegebenenfalls substituiertes Alkyl, Halogen, Nitro, Cyano, Alkylsulfonyl, Arylsulfonyl, Hydroxy, Alkoxy, Acyloxy stehen und $R^2$ und $R^3$ gleich oder verschieden sein können, mit der Einschränkung, daß $R^2$ oder $R^3$ nicht Brom, oder $R^2$ und $R^3$ nicht gleichzeitig Wasserstoff bedeuten,

X für Alkylen (mit 2 bis 4 C-Atomen), das einfach oder mehrfach durch Alkyl, Alkoxy, Aryl, Fluor oder Alkoxycarbonyl, eine Methylengruppe der Alkylenbrücke kann auch durch O, S, $SO_2$, SO, NH oder N-Alkyl ersetzt sein, substituiert sein kann,

steht.

2. Verbindungen der allgemeinen Formel I

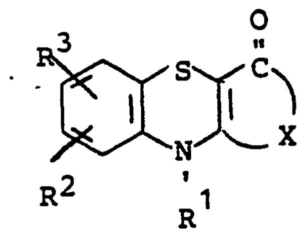

I

worin

$R_1$ für Wasserstoff und gegebenenfalls substituiertes Alkyl steht,

$R_2$, $R_3$ für Wasserstoff, gegebenenfalls substituiertes Alkyl, Halogen, Nitro, Cyano, Alkylsulfonyl, Arylsulfonyl, Hydroxy, Alkoxy, Acyloxy und

X für Alkylen (mit 2 bis 4 C-Atomen), das einfach oder mehrfach durch Alkyl, Alkoxy, Aryl, Fluor oder Alkoxycarbonyl substituiert sein kann, eine Methylgruppe der Alkylenbrücke kann auch durch O, S, $SO_2$, SO, NH oder N-Alkyl ersetzt sein,

steht, zur Verwendung bei der Bekämpfung von Krankheiten.

3. Verbindungen der allgemeinen Formel I gemäß Anspruch 2 zur Verwendung als Lipoxygenasehemmer, Antiphlogistika, Antimetastatika, Antirheumatika, Antiarthrotika Antiasthmatika, Antiallergika und/oder Gastroprotektiva.

4. Arzneimittel enthaltend mindestens eine Verbindung der allgemeinen Formel I gemäß Anspruch 2.

5. Verwendung von Verbindungen der allgemeinen Formel I gemäß Anspruch 2 bei der Bekämpfung von Krankheiten.

6. Verwendung von Verbindungen der allgemeinen Formel I gemäß Anspruch 2 als Lipoxygenasehemmer, Entzündungshemmer, Antimetastatika, Antiphlogistika, Antirheumatika, Antiatherosklerotika, Antithrombotika, Antiarthrotika, Antiasthmatika, Antiallergika, Gastroprotektiva und/oder bei kardiovakulären und/oder arebralen Erkrankungen.

7. Verfahren zur Herstellung von Arzneimitteln enthaltend mindestens eine Verbindung der allgemeinen Formel I gemäß Anspruch 2, dadurch gekennzeichnet, daß man Verbindungen der allgemeinen Formel I gemäß Anspruch 2 gegebenenfalls unter Verwendung von pharmazeutisch üblichen Hilfs- und Trägerstoffen in eine geeignete Applikationsform überführt.

8. Verfahren zur Herstellung von Verbindungen der allgemeinen Formel I gemäß Anspruch 1 und 2, dadurch gekennzeichnet, daß man 2-Aminothiophenole der Formel II

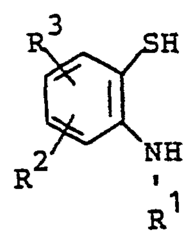

II

mit Verbindungen der Formel III

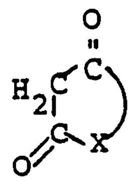

III

in Dimethylsulfoxid zur Reaktion bringt.

9. Verfahren zur Herstelllung von Verbindungen der allgemeinen Formel I gemäß Anspruch 1 und 2, dadurch gekennzeichnet, daß man Disulfide der allgemeinen Formel IV

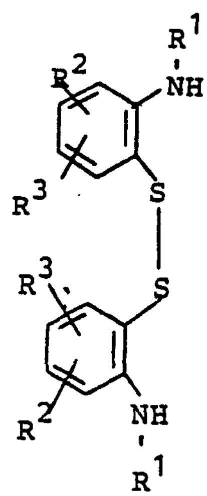

IV

mit Dicarbonylverbindungen der allgemeinen Formel III

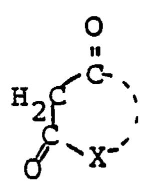

III

gegebenenfalls unter Verwendung katalytischer Mengen von Säuren zur Reaktion bringt.

0101898

Europäisches Patentamt

# EUROPÄISCHER RECHERCHENBERICHT

Nummer der Anmeldung

EP 83 10 7175

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CHEMICAL ABSTRACTS, Band 72, Nr. 13, 30. März 1970, Seite 406, Nr. 66965m, Columbus, Ohio, USA & JP - A - 69 27 588 (FUJISAWA PHARMACEUTICAL CO., LTD.) 15.11.1969 * Zusammenfassung * --- | 1-8 | C 07 D 279/20<br>C 07 D 279/16<br>C 07 D 513/04<br>A 61 K 31/54 //<br>(C 07 D 513/04<br>C 07 D 333/00<br>C 07 D 279/00 )<br>(C 07 D 513/04<br>C 07 D 307/00<br>C 07 D 279/00 ) |
| X | CHEMICAL ABSTRACTS, Band 78, Nr. 7, 19. Februar 1973, Seite 485, Nr. 43395k, Columbus, Ohio, USA V.I. SHVEDOV et al.: "Synthesis and reactions of 1,2,3,4-tetrahydrophenothiazines" & KHIM. GETEROTSIKL. SOEDIN. 1972, (11), 1509-13 * Zusammenfassung * --- | 1,8 | |
| X | JOURNAL OF THE CHEMICAL SOCIETY, PERKIN TRANSACTIONS I, Band (10), 1976, Seiten 1146-1149 S. MIYANO et al.: "Reactions of enamino-ketones. Part II. Synthesis of 4H-1,4-benzothiazines" * Seite 1147, oben, Formel 8; Seite 1149, Spalte 1, Absatz 2 * --- | 1,8 | **RECHERCHIERTE SACHGEBIETE (Int. Cl. 3)**<br>C 07 D 279/00<br>C 07 D 513/00<br>A 61 K 31/00 |
| X | JOURNAL OF THE CHEMICAL SOCIETY, PERKIN TRANSACTIONS I, No. 3, März 1982, Seite 831-834 T.L. GILCHRIST et al.: "1H-1,4-benzothiazines. New cyclic sulphonium ylides" * Seite 832, Formel 11 * --- -/- | 1,8 | |

Der vorliegende Recherchenbericht wurde für alle Patentansprüche erstellt.

| Recherchenort | Abschlußdatum der Recherche | Prüfer |
|---|---|---|
| DEN HAAG | 01-11-1983 | CHOULY J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0101898

Europäisches Patentamt

# EUROPÄISCHER RECHERCHENBERICHT

Nummer der Anmeldung

EP 83 10 7175

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-B-1 151 802 (PROMONTA)<br>* Spalte 3; Absatz 3; Spalte 4, Ansprüche *<br>--- | 1 | |
| A | DE-B-1 088 055 (PROMONTA)<br>* Ansprüche *<br>----- | 1,8 | |
| | | | RECHERCHIERTE SACHGEBIETE (Int. Cl. 3) |

Der vorliegende Recherchenbericht wurde für alle Patentansprüche erstellt.

| Recherchenort | Abschlußdatum der Recherche | Prüfer |
|---|---|---|
| DEN HAAG | 01-11-1983 | CHOULY J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82